# EUROPEAN PATENT SPECIFICATION

(11) **EP 4 025 915 B1**
(45) Date of publication and mention of the grant of the patent: **26.11.2025**
(21) Application number: 20767782.4
(22) Date of filing: 03.09.2020
(51) Int. Cl.: G01N 33/68, C07K 16/18

(54) **ASSAY FOR ASSESSING HEART FAILURE**
TEST ZUR BEURTEILUNG VON HERZVERSAGEN
DOSAGE POUR ÉVALUER UNE INSUFFISANCE CARDIAQUE

(30) Priority: 06.09.2019 GB 201912856
(43) Date of publication of application: 13.07.2022
(73) Proprietor: Nordic Bioscience A/S, 2730 Herlev (DK)
(72) Inventor: SUN, Shu, 2720 Vanløse (DK); REESE-PETERSEN, Alexander, Lynge, 3540 Lynge (DK); GENOVESE, Federica, 2000 Frederiksberg (DK); KARSDAL, Morten, 2100 København Ø (DK)
(74) Representative: Beck Greener LLP
(86) International application number: PCT/EP2020/074685
(87) International publication number: WO 2021/043945

(56) References cited:
- WO-A1-2010/124821
- US-A1- 2017 363 620
- JAVIER BARALLOBRE-BARREIRO ET AL: "Proteomics Analysis of Cardiac Extracellular Matrix Remodeling in a Porcine Model of Ischemia/Reperfusion Injury", CIRCULATION, vol. 125, no. 6, 14 February 2012 (2012-02-14), US, pages 789 - 802, XP055751328, ISSN: 0009-7322, DOI: 10.1161/CIRCULATIONAHA.111.056952
- GEBAUER JAN M ET AL: "Structure, evolution and expression of collagen XXVIII: Lessons from the zebrafish", MATRIX BIOLOGY, ELSEVIER, NL, vol. 49, 31 July 2015 (2015-07-31), pages 106 - 119, XP029432439, ISSN: 0945-053X, DOI: 10.1016/J.MATBIO.2015.07.001
- VEIT GUIDO ET AL: "Collagen XXVIII, a novel von Willebrand factor A domain-containing protein with many imperfections in the collagenous domain", JOURNAL OF BIOLOGICAL CHEMISTRY, AMERICAN SOCIETY FOR BIOCHEMISTRY AND MOLECULAR BIOLOGY, US, vol. 281, no. 6, 1 February 2006 (2006-02-01), pages 3494 - 3504, XP002434671, ISSN: 0021-9258, DOI: 10.1074/JBC.M509333200

## Description

### Field of the Invention

The present invention relates to immunoassays, in particular immunoassays for detecting and/or monitoring heart failure with preserved ejection fraction (HFpEF) in a patient, and monoclonal antibodies for use in said assays.

### Background

The burden of heart failure (HF) has increased dramatically over the last several years^{1,2}. Approximately half of HF is secondary to HF with preserved ejection fraction (HFpEF), which is anticipated to represent an even larger proportion of the total burden of HF as the population ages³. Despite multiple phase-III randomized controlled trials over the last few decades, a pharmacologic intervention proven to provide a clear benefit for this patient population remains to be identified.

The heterogeneity of the HFpEF syndrome has been identified as an important barrier to demonstrating the effectiveness of candidate pharmacologic interventions. Given the heterogenous nature of HFpEF, different degrees of contribution from various pathophysiological processes may unfavorably influence average responses to pharmacologic therapies tested in clinical trials. Therefore, the availability of simple, non-invasive biomarkers capable of readily identifying relevant underlying specific biologic processes that can be targeted with pharmacologic interventions represents a promising approach to enhance our clinical and therapeutic approach to HFpEF⁴.

The hetererogeneity of HFpEF also has important implications for the differential prognosis of individual patients. The ability to more effectively risk-stratify HFpEF patients is greatly needed. Novel risk-stratification markers may not only improve our ability to prognosticate HFpEF patients in clinical practice, but would be of great value to inform enrollment of high-risk individuals in future trials.

Myocardial fibrosis is thought to play a role in the pathophysiology of HFpEF^{5,6}. Increased fibrosis results from an excess of formation relative to degradation of collagen, ultimately leading increased interstitial collagen deposition in the interstitium. Increased myocardial extracellular matrix deposition has been demonstrated in HFpEF in autopsy specimens and *in vivo* studies⁶⁻⁸ and has been shown to correlate with LV passive stiffening and diastolic dysfunction in this condition^{5,8}. Myocardial fibrosis may also contribute to reduced coronary flow reserve^{6,9}, ventricular dyssynchrony and a propensity to arrhythmia^{10,11}. Given the role of myocardial fibrosis in HFpEF, simple fibrotic biomarkers that reflect the underlying dynamic process of fibrosis progression or regression of fibrosis would be highly valuable¹⁰.

The extracellular volume fraction (ECVF), an index of myocardial fibrosis measured by cardiac magnetic resonance imaging, has been reported to predict adverse outcomes in patients with HFpEF ^{12,13}, or at risk for HFpEF¹⁴. Although MRI will likely have an important role in the assessment of myocardial fibrosis in preclinical studies, early-phase research in humans and some clinical settings, its cost and availability are likely to limit or preclude its use in global phase-III trials and in clinical practice. In addition, many patients with HFpEF are not candidates for ECVF measurements due to claustrophobia or advanced renal disease. Thus, the search for circulating biomarkers of tissue fibrosis remains an area of great interest. The search for suitable biomarkers for HFpEF is, however, complicated by the notion that "fibrosis is not just fibrosis", and that ECM remodeling of different compartments and collagen types may have different biologic and prognostic implications¹⁵. For instance, differential associations of collagen neoepitope fragments and liver fibrosis has been reported in chronic hepatitis B vs. hepatitis C. Moreover, although myocardial fibrosis is thought to be important in HFpEF, extracardiac fibrosis may also play an important role. For example, fibrofatty infiltration of skeletal muscle has been reported in HFpEF¹⁶. Similarly, fibrosis may also occur in the arterial wall, the kidney and the liver dysfunction, all of which may contribute to adverse outcomes in this population.

WO2010/124821 teaches an immunoassay for assessing heart failure by measuring in a sample obtained from an individual the concentration of the marker endostatin, a C-terminal fragment of collagen XVIII.

Type XXVIII collagen is poorly described in literature but research entailing its physical role is slowly emerging. It is mainly located in peripheral nerves and dorsal root ganglia, but is also found in the skin^{17,18}. Type XXVIII collagen is a beaded collagen that structurally resembles type VI collagen, with two von Willebrand factor A domains flanking a 528 amino acid collagenous domain¹⁹. Type XXVIII collagen was found in very low levels in healthy lung tissue but was overexpressed in bleomycin-induced lung injury²⁰, which could indicate that cells expressing type XXVIII collagen might be involved in tissue repair processes. Type XXVIII collagen has also previously been seen to be upregulated in mouse hepatocarcinoma²¹.

### Summary

The present inventors have now determined that collagen type XXVIII formation is upregulated in HFpEF, and have developed a new competitive ELISA utilizing monoclonal antibodies targeting the C-terminal end of type XXVIII collagen.

Accordingly, in a first aspect the present invention provides a method of immunoassay for detecting and/or monitoring heart failure with a preserved ejection fraction (HFpEF) in a patient. The method comprises:
(i) contacting a biofluid sample from a patient with a monoclonal antibody that specifically binds to a C-terminal epitope of type XXVIII collagen, wherein said monoclonal antibody specifically binds to a C-terminus amino acid sequence QETCIQG (SEQ ID NO: 1),
(ii) detecting and determining the amount of binding between said monoclonal antibody and peptides in the sample, and
(iii) correlating said amount of binding of said monoclonal antibody as determined in step (ii) with values associated with normal healthy subjects and/or values associated with known disease severity and/or values obtained from said patient at a previous time point and/or a predetermined cut-off value.

The immunoassay may be, but is not limited to, a competition assay or a sandwich assay. The immunoassay may, for example, be a radioimmunoassay or an enzyme-linked immunosorbent assay (ELISA). Such assays are techniques known to the person skilled in the art.

The patient biofluid sample may be, but is not limited to, blood, serum, plasma, urine or a supernatant from cell or tissue cultures. Preferably the biofluid is serum or plasma, most preferably serum.

As used herein the term "monoclonal antibody" refers to both whole antibodies and to fragments thereof that retain the binding specificity of the whole antibody, such as for example a Fab fragment, F(ab')2 fragment, single chain Fv fragment, or other such fragments known to those skilled in the art. As is well known, whole antibodies typically have a "Y-shaped" structure of two identical pairs of polypeptide chains, each pair made up of one "light" and one "heavy" chain. The N-terminal regions of each light chain and heavy chain contain the variable region, while the C-terminal portions of each of the heavy and light chains make up the constant region. The variable region comprises three complementarity determining regions (CDRs), which are primarily responsible for antigen recognition. The constant region allows the antibody to recruit cells and molecules of the immune system. Antibody fragments retaining binding specificity comprise at least the CDRs and sufficient parts of the rest of the variable region to retain said binding specificity.

In the methods of the present invention, a monoclonal antibody comprising any constant region known in the art can be used. Human constant light chains are classified as kappa and lambda light chains. Heavy constant chains are classified as mu, delta, gamma, alpha, or epsilon, and define the antibody's isotype as IgM, IgD, IgG, IgA, and IgE, respectively. The IgG isotype has several subclasses, including, but not limited to IgGl, IgG2, IgG3, and IgG4. The monoclonal antibody may preferably be of the IgG isotype, including any one of IgGl, IgG2, IgG3 or IgG4.

The CDR of an antibody can be determined using methods known in the art such as that described by Kabat et al. Antibodies can be generated from B cell clones as described in the examples. The isotype of the antibody can be determined by ELISA specific for human IgM, IgG or IgA isotype, or human IgG1, IgG2, IgG3 or IgG4 subclasses. The amino acid sequence of the antibodies generated can be determined using standard techniques. For example, RNA can be isolated from the cells, and used to generate cDNA by reverse transcription. The cDNA is then subjected to PCR using primers which amplify the heavy and light chains of the antibody. For example primers specific for the leader sequence for all VH (variable heavy chain) sequences can be used together with primers that bind to a sequence located in the constant region of the isotype which has been previously determined. The light chain can be amplified using primers which bind to the 3' end of the Kappa or Lamda chain together with primers which anneal to the V kappa or V lambda leader sequence. The full length heavy and light chains can be generated and sequenced.

As noted above, in the methods according to the first aspect of the invention the biofluid sample is contacted with a monoclonal antibody which specifically binds to the C-terminus amino acid sequence QETCIQG (SEQ ID NO: 1) (also referred to herein as "PRO-C28"). Preferably said monoclonal antibody does not recognize or specifically bind to an elongated version of said C-terminus amino acid sequence which is QETCIQGA (SEQ ID NO: 2). Preferably said monoclonal antibody does not recognize or specifically bind to a truncated version of said C-terminus amino acid sequence which is QETCIQ (SEQ ID NO: 3).

Preferably, the ratio of the affinity of said antibody for the C-terminus amino acid sequence QETCIQG (SEQ ID NO: 1) to the affinity of said antibody for the elongated C-terminus amino acid sequence QETCIQGA (SEQ ID NO: 2) is at least 10 to 1, and more preferably is at least 50 to 1, at least 100 to 1, at least 500 to 1, at least 1,000 to 1, at least 10,000 to 1, at least 100,000 to 1, or at least 1,000,000 to 1.

Preferably, the ratio of the affinity of said antibody for the C-terminus amino acid sequence QETCIQG (SEQ ID NO: 1) to the affinity of said antibody for the truncated C-terminus amino acid sequence QETCIQ (SEQ ID NO: 3) is at least 10 to 1, and more preferably is at least 50 to 1, at least 100 to 1, at least 500 to 1, at least 1,000 to 1, at least 10,000 to 1, at least 100,000 to 1, or at least 1,000,000 to 1.

As used herein the term "C-terminus" refers to a C-terminal peptide sequence at the extremity of a polypeptide, i.e. at the C-terminal end of the polypeptide, and is not to be construed as meaning in the general direction thereof.

Monoclonal antibodies that specifically bind to the C-terminus amino acid sequence QETCIQG (SEQ ID NO: 1) can be generated via any suitable techniques known in the art. For example, the monoclonal antibody may be raised against a synthetic peptide having the amino acid sequence QETCIQG (SEQ ID NO: 1), such as for example by: immunizing a rodent (or other suitable mammal) with a synthetic peptide consisting of the sequence QETCIQG (SEQ ID NO: 1), which optionally may linked to an immunogenic carrier protein (such as keyhole limpet hemocyanin), isolating and cloning a single antibody producing cell, and assaying the resulting monoclonal antibodies to ensure that they have the desired specificity. An exemplary protocol for producing a monoclonal antibody that that specifically bind to the C-terminus amino acid sequence QETCIQG (SEQ ID NO: 1) is described infra.

In some embodiments of the methods according to the first aspect of the invention, the amount of binding of the monoclonal antibody specific for the C-terminal epitope of type XXVIII collagen is correlated with values associated with normal healthy subjects and/or with values associated with known disease severity and/or with values obtained from the patient at a previous point in time.

As used herein the term "values associated with normal healthy subjects and/or values associated with known disease severity" means standardised quantities determined by the method described supra for subjects considered to be healthy, i.e. without a cardiovascular disease, and/or standardised quantities determined by the method described supra for subjects known to have a cardiovascular disease (i.e. HFpEF) with a known severity.

In some embodiments of the method according to the first aspect, the amount of binding of the monoclonal antibody specific for the C-terminal epitope of type XXVIII collagen is correlated with one or more predetermined cut-off values.

As used herein the "cut-off value" means an amount of binding that is determined statistically to be indicative of a high likelihood of cardiovascular disease (i.e. HFpEF) in a patient, in that a measured value of biomarker binding in a patient sample that is at or above the statistical cutoff value corresponds to at least a 70% probability, preferably at least an 80% probability, preferably at least an 85% probability, more preferably at least a 90% probability, and most preferably at least a 95% probability of the presence or likelihood of cardiovascular disease (i.e. HFpEF).

The predetermined cut-off value for the amount of binding of the monoclonal antibody specific for the C-terminal epitope of type XXVIII collagen is preferably at least 100 ng/mL. In this regard, through the use of statistical analyses it has been found that a measured amount of binding of the monoclonal antibody specific for the C-terminal epitope of type XXVIII collagen of at least 100 ng/mL or greater may be determinative of cardiovascular disease (i.e. HFpEF). By having a statistical cut-off value of at least 100 ng/mL it is possible to utilise the method of the invention to give a diagnosis of cardiovascular disease (i.e. HFpEF) with a high level of confidence. Applying such statistical cut-off values are particularly advantageous as it results in a standalone diagnostic assay; i.e. it removes the need for any direct comparisons with healthy individuals and/or patients with known disease severity in order to arrive at a diagnostic conclusion. This may also be particularly advantageous when utilising the assay to evaluate patients that already have medical signs or symptoms that are generally indicative of cardiovascular disease (e.g. as determined by a physical examination and/or consultation with a medical professional) as it may act as a quick and definitive tool for corroborating the initial diagnosis and thus potentially remove the need for more invasive procedures, and expedite the commencement of a suitable treatment regimen. It may also avoid the need for a lengthy hospital stay. In the particular case of cardiovascular disease, an expedited conclusive diagnosis may result in the disease being detected at an earlier stage, which may in turn improve overall chances of survival, and/or reduce the risk of hospitalisation.

In a second aspect the present invention provides an immunoassay kit comprising a monoclonal antibody that specifically binds to a C-terminus amino acid sequence QETCIQG (SEQ IDNO: 1), and at least one of:
- a streptavidin coated well plate;
- a biotinylated peptide Biotin-L-QETCIQG (SEQ ID NO: 4), wherein L is an optional linker;
- a secondary antibody for use in a sandwich immunoassay;
- a calibrator peptide comprising the sequence QETCIQG (SEQ ID NO: 1);
- an antibody biotinylation kit;
- an antibody HRP labeling kit;
- an antibody radiolabeling kit; and
- an assay visualization kit.

The immunoassay kit is suitable for carrying out a method according to the first aspect, and accordingly preferred embodiments of the second aspect will be apparent from the above discussion of the preferred embodiments of the first aspect. For example, the kit is suitable for detecting and/or monitoring HFpEF in a patient. The monoclonal antibody that specifically binds to a C-terminus amino acid sequence QETCIQG (SEQ ID NO: 1) is preferably a monoclonal antibody that has been raised against a synthetic peptide having the amino acid sequence QETCIQG (SEQ ID NO: 1). Preferably said monoclonal antibody does not recognize or specifically bind to an elongated version of said C-terminus amino acid sequence which is QETCIQGA (SEQ ID NO: 2). Preferably said monoclonal antibody does not recognize or specifically bind to a truncated version of said C-terminus amino acid sequence which is QETCIQ (SEQ ID NO: 3).

In a third aspect, the present invention provides a monoclonal antibody that specifically binds to a C-terminus amino acid sequence QETCIQG (SEQ ID NO: 1). Preferred embodiments of the third aspect will be again be apparent from the above discussion of the preferred embodiments of the first aspect. For example, the monoclonal antibody is preferably a monoclonal antibody that has been raised against a synthetic peptide having the amino acid sequence QETCIQG (SEQ ID NO: 1). Preferably, the monoclonal antibody does not recognize or specifically bind to an elongated version of said C-terminus amino acid sequence which is QETCIQGA (SEQ ID NO: 2). Preferably, the monoclonal antibody does not recognize or specifically bind to a truncated version of said C-terminus amino acid sequence which is QETCIQ (SEQ ID NO: 3).

### Figures

Figure 1: Antibody specificity. Reactivity was tested using the standard peptide (QETCIQG) (SEQ ID NO: 1), an elongated peptide (QETCIQGA) (SEQ ID NO: 2), a non-sense peptide (GLRPGSEYTV) (SEQ ID NO: 7) and a non-sense coater (GLRPGSEYTV-K-Biotin) (SEQ ID NO: 8).
Figure 2: Levels of PRO-C28 in serum of healthy controls (HC) and patients with HFpEF.

### Examples

The presently disclosed embodiments are described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

In the following examples, the following materials and methods were employed.

### Materials and methods

All reagents used for the experiments were high quality standards from companies such as Sigma Aldrich (St. Louis, MO, USA) and Merck (Whitehouse Station, NJ, USA). The synthetic peptides used for immunization and assay development were purchased from the Genscript (New Jersey, USA). Human serum from healthy donors was purchased from a commercial supplier (Lee Biosolutions, MO 63043, USA). Human serum from HFpEF patients belonged to the TRAINING-HF cohort, obtained through an academic collaboration with the INCLIVA Health Research Institute in Valencia, Spain.

### Generation of monoclonal antibodies targeting PRO-C28

Monoclonal antibodies targeting the C-terminal end of collagen type XXVIII were generated by raising antibodies against the 7 amino acid sequence QETCIQG (SEQ ID NO: 1) ("PRO-C28") found at the C-terminus of collagen type XXVIII. This 7 amino acid sequence was chosen rather than a longer sequence, such as the 10 amino acid C-terminus sequence KECQETCIQG (SEQ ID NO: 5), in order to reduce the number of cystein residues and so avoid the formation of a Cys-cys bridge in in the immunogenic peptide that was being used to generate the antibodies.

The protocol used for generation of monoclonal antibodies targeting PRO-C28 was as follows.

Immunization of 6-7 week old female Balb/C mice (weighing 14-18g) was initiated by subcutaneous injection of 200 uL emulsified antigen solution comprising 100 ug immunogenic peptide (KLH-CGG-QETCIQG (SEQ ID NO: 6), where 'KHL' indicates keyhole limpet hemocyanin and CGG is a conjugation linker) in Stimune Immunogenic Adjuvant (SPECOL) (Cat #7925000, Invitrogen). The immunizations were repeated every 2^{nd} week until stable serum antibody titer levels were reached. The mouse with the highest serum titer and best inhibition was selected for fusion and rested for at least three weeks following the last immunization. Subsequently, the mouse was boosted intravenously with 100 ug immunogenic peptide in 100 uL 0.9% NaCl solution three days before isolation of the spleen for cell fusion. To produce hybridoma cells, the mouse spleen cells were fused with SP2/0 myeloma cells as described by Gefter et al. The hybridoma cells were cloned in culture dishes using the semi-solid medium method. The clones were then plated into 96-well microtiter plates for further growth, and the limiting dilution method was applied to promote monoclonal growth. Indirect ELISA performed on streptavidin-coated plates was used for the screening of supernatant reactivity. Biotin-QETCIQG was used as the screening peptide, while the standard peptide (QETCIQG (SEQ ID NO: 1)) an elongated peptide (QETCIQGA (SEQ ID NO: 2)), a non-sense peptide (GLRPGSEYTV (SEQ ID NO: 7)) and a non-sense coater (GLRPGSEYTV-K-Biotin (SEQ ID NO: 8)) were used for further testing of the specificity of the clones. Supernatant was collected from the hybridoma cells, and purified using HiTrap affinity columns (GE Healthcare Life Science, Little Chalfront, Buckinghamshire, UK) according to manufacturer's instructions. All animals were treated according to the guidelines for animal welfare.

### Clone selection and characterization

The best antibody producing hybridomas were screened for reactivity towards the standard peptide (QETCIQG (SEQ ID NO: 1)) in the competitive ELISA described below, and the clone showing the most reactivity was selected for production of monoclonal antibodies targeting PRO-C28. Antibody specificity was tested using the standard peptide (QETCIQG (SEQ ID NO: 1)), elongated peptide (QETCIQGA (SEQ ID NO: 2)), non-sense peptide (GLRPGSEYTV (SEQ ID NO: 7)) and non-sense coater (GLRPGSEYTV-K-Biotin (SEQ ID NO: 8)). The isotype of the monoclonal antibody was determined using the Clonotyping System-HRP kit, cat. 5300-05 (Southern Biotech, Birmingham, AL, USA).

### PRO-C28 ELISA

A 96-well streptavidin-coated ELISA plate from Roche, cat.11940279, was coated with 100 µL/well of the biotinylated peptide Biotin-QETCIQG (SEQ ID NO: 4) dissolved in assay buffer (25mM TBS-BTE + 2 g/l NaCl, pH 8), incubated for 30 min at 20°C in the dark with shaking, and subsequently washed 5 times in washing buffer (20 mM Tris, 50 mM NaCl, pH 7.2). Thereafter 20 µl of peptide calibrator or sample were added to appropriate wells, followed by 100 µl of purified antibody solution (monoclonal antibodies specific for PRO-C28 dissolved in assay buffer), and incubated for 1 hour at 20°C with shaking, followed by washing 5 times in washing buffer. Next, 100µL secondary antibody solution (horseradish peroxidase (HRP) labeled anti-mouse antibodies dissolved in the same assay buffer as used for the monoclonal antibody specific for PRO-C28) was added to each well, incubated for 1 hour at 20°C with shaking, followed by washing 5 times in washing buffer. Finally, 100 µl tetramethylbenzinidine (TMB) (Kem-En-Tec cat.: 438OH) was added to each well, the plate was incubated for 15 min at 20°C in the dark, and in order to stop the reaction 100 µl of stopping solution (1% H₂SO₄) was added and the plate was then analyzed in the ELISA reader at 450 nm with 650 nm as the reference (Molecular Devices, SpectraMax M, CA, USA). A calibration curve was plotted using a 4-parametric mathematical fit model.

### Technical evaluation of PRO-C28 ELISA

A twofold dilution of human serum, human urine and EDTA, heparin or citrate treated human plasma samples (four of each type of sample) was used to assess the linearity. The linearity was calculated as a percentage of recovery of the undiluted sample.

The intra- and inter-assay variation was determined by 10 independent runs of five quality control (QC) and two kit controls run in double determinations.

Accuracy of the assay was measured in healthy human serum samples spiked with standard peptide, and calculated as the percentage recovery of serum in buffer.

Lower limit of measurement range (LLMR) and upper limit of measurement range (ULMR) was calculated based on the 10 individual standard curves from the intra- and inter-assay variation.

### Biological validation of PRO-C28 as a biomarker for HFpEF

PRO-C28 was measured in serum samples in a cohort of patients with HFpEF (heart failure with preserved ejection fraction) and a cohort of healthy controls, using the PRO-C28 ELISA protocol described above. The patient demographics are shown in Table 1.

**Table 1: Patient demographics**

| | HFpEF | | HC | |
|---|---|---|---|---|
| | N | Mean (SD) | N | Mean (SD) |
| Age* | 43 | 73 (8) | 54 | 39 (11) |
| Gender (Male/Female) | 25/18 | | 24/30 | |
| NYHA (class II/class III) | 34/9 | | NA | NA |
| Ejection fraction | 43 | 67 (10) | NA | NA |
| Systolic blood pressure | 43 | 130 (14) | NA | NA |

| | | | | |
|---|---|---|---|---|
| *Age is significantly different between the 2 groups, however there is no correlation between age and PRO-C28 in either of the groups. | | | | |

### Results

### Clone selection and characterization

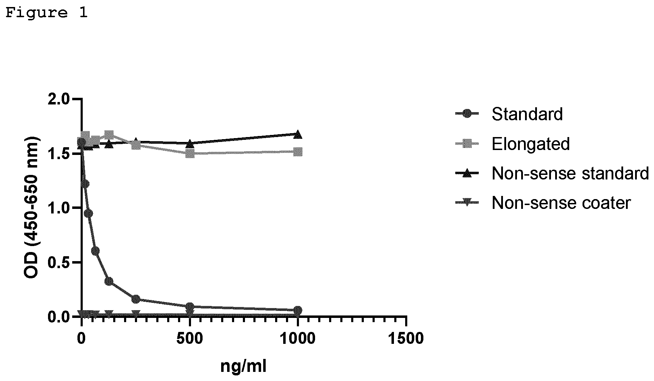

The best antibody producing hybridomas were screened for reactivity and selectivity towards the standard peptide, and based on reactivity the clone NBH218#65 8C11-2F10-1H7 was chosen and used for production of monoclonal antibodies targeting PRO-C28 for use the technical and biological evaluation of the PRO-C28 ELISA. The monoclonal antibodies were of the isotype: IgG2b, k. No reactivity was found towards the elongated peptide, non-sense peptide or non-sense coater (Figure 1).

### Technical evaluation of the PRO-C28 ELISA

A series of technical validations were performed to evaluate the PRO-C28 ELISA assay. A summary of the validation data is shown in Table 2.

**Table 2: technical characteristics of the PRO-C28 competitive ELISA**

| **Intra/inter variation** | Intra-variation: 6% |
|---|---|
| | Inter-variation: 14% |
| **Dilution recovery** | Serum: 112% (mean) |
| | EDTA plasma: 99% (mean) |
| | Heparin plasma: 88% (mean) |
| | Citrate plasma: 94% (mean) |
| | Urine: 99% (mean) |
| **Spiking recovery (peptide in serum)** | 92% (mean) |
| **Measurement range** | 9.5-282 ng/ml |
| **Ic50** | 53.2 ng/ml |

### Biological evaluation of PRO-C28 as a biomarker for HFpEF

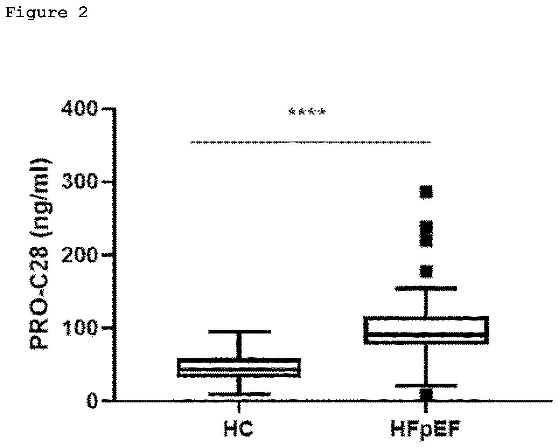

PRO-C28 levels were measured, using the PRO-C28 ELISA, in serum samples in a cohort of patients with heart failure with preserved ejection fraction (HFpEF) and in a cohort of healthy controls (HC). The levels of the biomarker in serum samples from the two cohorts were then compared, using Mann-Whitney test (non-paramteric data). The results are shown in Figure 2 (in which the results are presented as Tukey's boxplot). As can be seen, PRO-C28 was significantly elevated in the serum from HFpEF patients as compared to healthy controls (p<0.0001).

In addition, at three different time points over the course of the study, NT-proBNP levels were measured in the HFpEF patient serum samples, and the measured concentrations of NT-proBNP were then compared using a Spearman correlation with the measured concentrations of PRO-C28 (measured as discussed above) in the same sample. As shown below, in Table 3, it was found that the levels of PRO-C28 in the HFpEF cohort correlated significantly with NT-proBNP, which is the standard clinical assessment biomarker for diagnosing and monitoring HF.

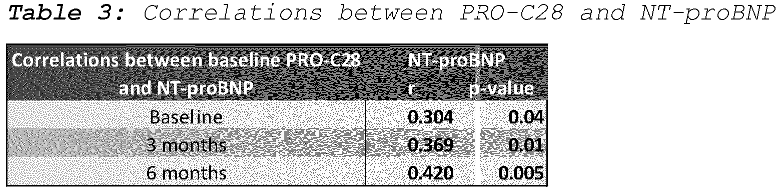

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

### References

1. Lam CS, Donal E, Kraigher-Krainer E, Vasan RS. Epidemiology and clinical course of heart failure with preserved ejection fraction. Eur J Heart Fail 2011;13:18-28.
2. Lloyd-Jones DM, Hong Y, Labarthe D et al. Defining and setting national goals for cardiovascular health promotion and disease reduction: the American Heart Association's strategic Impact Goal through 2020 and beyond. Circulation 2010;121:586-613.
3. Lam CS, Donal E, Kraigher-Krainer E, Vasan RS. Epidemiology and clinical course of heart failure with preserved ejection fraction. Eur J Heart Fail 2011;13:18-28.
4. Chirinos JA. Deep Phenotyping of Systemic Arterial Hemodynamics in HFpEF (Part 2): Clinical and Therapeutic Considerations. J Cardiovasc Transl Res 2017;10:261-274.
5. Rommel KP, von Roeder M, Latuscynski K et al. Extracellular Volume Fraction for Characterization of Patients With Heart Failure and Preserved Ejection Fraction. J Am Coll Cardiol 2016;67:1815-25.
6. Mohammed SF, Hussain S, Mirzoyev SA, Edwards WD, Maleszewski JJ, Redfield MM. Coronary microvascular rarefaction and myocardial fibrosis in heart failure with preserved ejection fraction. Circulation 2015;131:550-9.
7. Chirinos JA, Akers SR, Trieu L et al. Heart Failure, Left Ventricular Remodeling, and Circulating Nitric Oxide Metabolites. J Am Heart Assoc 2016;5.
8. Su MY, Lin LY, Tseng YH et al. CMR-verified diffuse myocardial fibrosis is associated with diastolic dysfunction in HFpEF. JACC Cardiovasc Imaging 2014;7:991-7.
9. Mohammed SF, Majure DT, Redfield MM. Zooming in on the Microvasculature in Heart Failure With Preserved Ejection Fraction. Circ Heart Fail 2016;9.
10. Richards AM. Circulating Biomarkers of Cardiac Fibrosis: Do We Have Any and What Use Are They? Circ Heart Fail 2017;10.
11. Lin LY, Wu CK, Juang JM et al. Myocardial Regional Interstitial Fibrosis is Associated With Left IntraVentricular Dyssynchrony in Patients With Heart Failure: A Cardiovascular Magnetic Resonance Study. Sci Rep 2016;6:20711.
12. Duca F, Kammerlander AA, Zotter-Tufaro C et al. Interstitial Fibrosis, Functional Status, and Outcomes in Heart Failure With Preserved Ejection Fraction: Insights From a Prospective Cardiac Magnetic Resonance Imaging Study. Circ Cardiovasc Imaging 2016;9.
13. Roy C, Slimani A, de Meester C et al. Associations and prognostic significance of diffuse myocardial fibrosis by cardiovascular magnetic resonance in heart failure with preserved ejection fraction. J Cardiovasc Magn Reson 2018;20:55.
14. Schelbert EB, Fridman Y, Wong TC et al. Temporal Relation Between Myocardial Fibrosis and Heart Failure With Preserved Ejection Fraction: Association With Baseline Disease Severity and Subsequent Outcome. JAMA Cardiol 2017.
15. Nielsen MJ, Karsdal MA, Kazankov K et al. Fibrosis is not just fibrosis - basement membrane modelling and collagen metabolism differs between hepatitis B- and C-induced injury. Aliment Pharmacol Ther 2016;44:1242-1252.
16. Haykowsky MJ, Kouba EJ, Brubaker PH, Nicklas BJ, Eggebeen J, Kitzman DW. Skeletal muscle composition and its relation to exercise intolerance in older patients with heart failure and preserved ejection fraction. Am J Cardiol 2014;113:1211-6.
17. Gebauer, J. M., Kobbe, B., Paulsson, M. & Wagener, R. Structure, evolution and expression of collagen XXVIII: Lessons from the zebrafish. Matrix Biol. 49, 106-119 (2016).
18. Veit, G. et al. Collagen XXVIII, a novel von Willebrand factor A domain-containing protein with many imperfections in the collagenous domain. J. Biol. Chem. 281, 3494-3504 (2006).
19. Annis, D. S., Mosher, D. F. & Roberts, D. D. NIH Public Access. 27, 339-351 (2009).
20. Schiller, H. B. et al. Time- and compartment-resolved proteome profiling of the extracellular niche in lung injury and repair. Mol. Syst. Biol. 11, 819-819 (2015).
21. Lai KKY, Shang S, Lohia N, Booth GC, Masse DJ, Fausto N, et al. (2011) Extracellular Matrix Dynamics in Hepatocarcinogenesis: a Comparative Proteomics Study of PDGFC Transgenic and Pten Null Mouse Models. PLoS Genet 7(6): e1002147. https://doi.org/10.1371/journal.pgen.1002147

## Claims

1. A method of immunoassay for detecting and/or monitoring heart failure with a preserved ejection fraction in a patient, the method comprising:
(i) contacting a biofluid sample from a patient with a monoclonal antibody that specifically binds to a C-terminal epitope of type XXVIII collagen, wherein said monoclonal antibody specifically binds to a C-terminus amino acid sequence QETCIQG (SEQ ID NO: 1),
(ii) detecting and determining the amount of binding between said monoclonal antibody and peptides in the sample, and.
(iii) correlating said amount of binding of said monoclonal antibody as determined in step (ii) with values associated with normal healthy subjects and/or values associated with known disease severity and/or values obtained from said patient at a previous time point and/or a predetermined cut-off value.

2. The method of claim 1, wherein said monoclonal antibody does not recognize or specifically bind to an elongated version of said C-terminus amino acid sequence which is QETCIQGA (SEQ ID NO: 2).

3. The method of claim 1 or 2, wherein said monoclonal antibody does not recognize or specifically bind to a truncated version of said C-terminus amino acid sequence which is QETCIQ (SEQ ID NO: 3).

4. The method of any one of claims 1-3, wherein the monoclonal antibody is raised against a synthetic peptide having the amino acid sequence QETCIQG (SEQ ID NO: 1).

5. The method of any one of claims 1-4, wherein said biofluid is blood, serum, plasma, urine or a supernatant from cell or tissue cultures.

6. The method of any one of claims 1-5, wherein said immunoassay is a competition assay or a sandwich assay.

7. The method of any one of claims 1-6, wherein said immunoassay is a radioimmunoassay or an enzyme-linked immunosorbent assay.

8. An immunoassay kit comprising a monoclonal antibody that specifically binds to a C-terminus amino acid sequence QETCIQG (SEQ ID NO: 1), and at least one of:
- a streptavidin coated well plate;
- a biotinylated peptide Biotin-L-QETCIQG (SEQ ID NO: 4), wherein L is an optional linker;
- a secondary antibody for use in a sandwich immunoassay;
- a calibrator peptide comprising the sequence QETCIQG (SEQ ID NO: 1);
- an antibody biotinylation kit;
- an antibody HRP labeling kit;
- an antibody radiolabeling kit; and
- an assay visualization kit.

9. An immunoassay kit as claimed in claim 8, wherein the monoclonal antibody is raised against a synthetic peptide having the amino acid sequence QETCIQG (SEQ ID NO: 1).

10. An assay kit as claimed in claim 8 or 9, wherein the monoclonal antibody does not recognize or specifically bind to an elongated version of said C-terminus amino acid sequence which is QETCIQGA (SEQ ID NO: 2).

11. An assay kit as claimed in any one of claims 8-10, wherein the monoclonal antibody does not recognize or specifically bind to a truncated version of said C-terminus amino acid sequence which is QETCIQ (SEQ ID NO: 3).

12. A monoclonal antibody that specifically binds to a C-terminus amino acid sequence QETCIQG (SEQ ID NO: 1).

13. The monoclonal antibody of claim 12, wherein the monoclonal antibody is raised against a synthetic peptide having the amino acid sequence QETCIQG (SEQ ID NO: 1).

14. The monoclonal antibody of claim 12 or 13, wherein the monoclonal antibody does not recognize or specifically bind to an elongated version of said C-terminus amino acid sequence which is QETCIQGA (SEQ ID NO: 2).

15. The monoclonal antibody of any one of claims 12-14, wherein the monoclonal antibody does not recognize or specifically bind to a truncated version of said C-terminus amino acid sequence which is QETCIQ (SEQ ID NO: 3).

## Patentansprüche

1. Ein Verfahren zum Immunoassay zum Nachweis und/oder zur Überwachung von Herzinsuffizienz mit erhaltener Auswurffraktion bei einem Patienten, umfassend:
(i) das Kontaktieren einer Bioflüssigkeitsprobe von einem Patienten mit einem monoklonalen Antikörper, der spezifisch an ein C-terminales Epitop des Kollagen Typs XXVIII bindet, wobei besagter monoklonaler Antikörper spezifisch an eine C-terminale Aminosäuresequenz QETCIQG (SEQ ID Nr. 1) bindet,
(ii) das Erkennen und Bestimmen der Menge der Bindung zwischen besagtem monoklonalen Antikörper und Peptiden in der Probe, und
(iii) die Korrelation der in Schritt (ii) bestimmten Menge der Bindung des monoklonalen Antikörpers mit Werten, die mit normalen gesunden Probanden assoziiert sind und/oder mit Werten, die mit bekannter Krankheitsschwere assoziiert sind und/oder mit Werten, die von besagtem Patienten zu einem früheren Zeitpunkt erhalten wurden und/oder mit einem vorbestimmten Grenzwert.

2. Ein Verfahren nach Anspruch 1, wobei besagter monoklonaler Antikörper keine verlängerte Version der C-terminalen Aminosäuresequenz, welche QETCIQGA (SEQ ID Nr. 2) ist, erkennt oder spezifisch bindet.

3. Ein Verfahren nach Anspruch 1 oder 2, wobei besagter monoklonaler Antikörper keine verkürzte Version der C-terminalen Aminosäuresequenz, welche QETCIQ (SEQ ID Nr. 3) ist, erkennt oder spezifisch bindet.

4. Ein Verfahren nach einem der Ansprüche 1 bis 3, wobei der monoklonale Antikörper gegen ein synthetisches Peptid mit der Aminosäuresequenz QETCIQG (SEQ ID Nr. 1) erzeugt wurde.

5. Ein Verfahren nach einem der Ansprüche 1 bis 4, wobei die Bioflüssigkeitsprobe Blut, Serum, Plasma, Urin oder ein Überstand aus Zell- oder Gewebekulturen ist.

6. Ein Verfahren nach einem der Ansprüche 1 bis 5, wobei der Immunoassay ein Konkurrenzassay oder ein Sandwichassay ist.

7. Ein Verfahren nach einem der Ansprüche 1 bis 6, wobei der Immunoassay ein Radioimmunoassay oder ein enzymgekoppelter Immunadsorptionstest (ELISA) ist.

8. Ein Immunoassay-Kit umfassend einen monoklonalen Antikörper, der spezifisch an die C-terminale Aminosäuresequenz QETCIQG (SEQ ID Nr. 1) bindet, und mindestens eines der folgenden:
- eine Streptavidin-beschichtete Mikrotiterplattenvertiefung;
- ein biotinyliertes Peptid Biotin-L-QETCIQG (SEQ ID Nr. 4), wobei L ein optionaler Linker ist;
- einen Sekundärantikörper zur Verwendung in einem Sandwichimmunoassay;
- ein Kalibrationspeptid mit der Sequenz QETCIQG (SEQ ID Nr. 1);
- ein Antikörper-Biotinylerungskit;
- ein Antikörper-HRP-Markierungskit;
- ein Antikörper-Radiomarkierungskit; und
- ein Kit zur Darstellung des Assays.

9. Ein Immunoassay-Kit nach Anspruch 8, wobei der monoklonale Antikörper gegen ein synthetisches Peptid mit der Aminosäuresequenz QETCIQG (SEQ ID Nr. 1) erzeugt wurde.

10. Ein Assay-Kit nach Anspruch 8 oder 9, wobei der monoklonale Antikörper keine verlängerte Version der C-terminalen Aminosäuresequenz, welche QETCIQGA (SEQ ID Nr. 2) ist, erkennt oder spezifisch bindet.

11. Ein Assay-Kit nach einem der Ansprüche 8 bis 10, wobei der monoklonale Antikörper keine verkürzte Version der C-terminalen Aminosäuresequenz, welche QETCIQ (SEQ ID Nr. 3) ist, erkennt oder spezifisch bindet.

12. Ein monoklonaler Antikörper, der spezifisch an die C-terminale Aminosäuresequenz QETCIQG (SEQ ID Nr. 1) bindet.

13. Ein monoklonaler Antikörper nach Anspruch 12, wobei der Antikörper gegen ein synthetisches Peptid mit der Aminosäuresequenz QETCIQG (SEQ ID Nr. 1) erzeugt wurde.

14. Ein monoklonaler Antikörper nach Anspruch 12 oder 13, wobei der Antikörper keine verlängerte Version der C-terminalen Aminosäuresequenz, welche QETCIQGA (SEQ ID Nr. 2) ist, erkennt oder spezifisch bindet.

15. Ein monoklonaler Antikörper nach einem der Ansprüche 12 bis 14, wobei der Antikörper keine verkürzte Version der C-terminalen Aminosäuresequenz, welche QETCIQ (SEQ ID Nr. 3) ist, erkennt oder spezifisch bindet.

## Revendications

1. Procédé de dosage immunologique pour détecter et/ou surveiller une insuffisance cardiaque avec une fraction d'éjection préservée chez un patient, le procédé comprenant :
(i) la mise en contact d'un échantillon de fluide biologique provenant d'un patient avec un anticorps monoclonal qui se lie spécifiquement à un épitope C-terminal du collagène de type XXVIII, où ledit anticorps monoclonal se lie spécifiquement à une séquence d'acides aminés C-terminale QETCIQG (SEQ ID NO : 1),
(ii) la détection et la détermination de la quantité de liaison entre ledit anticorps monoclonal et les peptides dans l'échantillon, et
(iii) la corrélation de ladite quantité de liaison dudit anticorps monoclonal telle que déterminée à l'étape (ii) avec des valeurs associées à des sujets sains normaux et/ou des valeurs associées à une gravité connue de la maladie et/ou des valeurs obtenues à partir dudit patient à un moment antérieur et/ou une valeur seuil prédéterminée.

2. Procédé selon la revendication 1, où ledit anticorps monoclonal ne reconnaît pas ou ne se lie pas spécifiquement à une version allongée de ladite séquence d'acides aminés C-terminale qui est QETCIQGA (SEQ ID NO : 2).

3. Procédé selon la revendication 1 ou 2, où ledit anticorps monoclonal ne reconnaît pas ou ne se lie pas spécifiquement à une version tronquée de ladite séquence d'acides aminés C-terminale qui est QETCIQ (SEQ ID NO : 3).

4. Procédé selon l'une quelconque des revendications 1 à 3, où l'anticorps monoclonal est produit contre un peptide synthétique ayant la séquence d'acides aminés QETCIQG (SEQ ID NO : 1).

5. Procédé selon l'une quelconque des revendications 1 à 4, où ledit fluide biologique est du sang, du sérum, du plasma, de l'urine ou un surnageant provenant de cultures cellulaires ou tissulaires.

6. Procédé selon l'une quelconque des revendications 1 à 5, où ledit dosage immunologique est un dosage par compétition ou un dosage en sandwich.

7. Procédé selon l'une quelconque des revendications 1 à 6, où ledit dosage immunologique est un dosage radio-immunologique ou un dosage d'immunoabsorption enzymatique.

8. Kit de dosage immunologique comprenant un anticorps monoclonal qui se lie spécifiquement à une séquence d'acides aminés C-terminale QETCIQG (SEQ ID NO : 1), et au moins l'un des éléments suivants :
- une plaque à puits recouverte de streptavidine ;
- un peptide biotinylé Biotine-L-QETCIQG (SEQ ID NO : 4), où L est un lieur facultatif ;
- un anticorps secondaire destiné à être utilisé dans un dosage immunologique en sandwich ;
- un peptide calibrateur comprenant la séquence QETCIQG (SEQ ID NO : 1) ;
- un kit de biotinylation d'anticorps ;
- un kit de marquage HRP d'anticorps ;
- un kit de radiomarquage d'anticorps ; et
- un kit de visualisation de dosage.

9. Kit de dosage immunologique selon la revendication 8, où l'anticorps monoclonal est produit contre un peptide synthétique ayant la séquence d'acides aminés QETCIQG (SEQ ID NO : 1).

10. Kit de dosage selon la revendication 8 ou 9, où l'anticorps monoclonal ne reconnaît pas ou ne se lie pas spécifiquement à une version allongée de ladite séquence d'acides aminés C-terminale qui est QETCIQGA (SEQ ID NO : 2).

11. Kit de dosage selon l'une quelconque des revendications 8 à 10, où l'anticorps monoclonal ne reconnaît pas ou ne se lie pas spécifiquement à une version tronquée de ladite séquence d'acides aminés C-terminale qui est QETCIQ (SEQ ID NO : 3).

12. Anticorps monoclonal qui se lie spécifiquement à une séquence d'acides aminés C-terminale QETCIQG (SEQ ID NO : 1).

13. Anticorps monoclonal selon la revendication 12, où l'anticorps monoclonal est produit contre un peptide synthétique ayant la séquence d'acides aminés QETCIQG (SEQ ID NO : 1).

14. Anticorps monoclonal selon la revendication 12 ou 13, où l'anticorps monoclonal ne reconnaît pas ou ne se lie pas spécifiquement à une version allongée de ladite séquence d'acides aminés C-terminale qui est QETCIQGA (SEQ ID NO : 2).

15. Anticorps monoclonal selon l'une quelconque des revendications 12 à 14, où l'anticorps monoclonal ne reconnaît pas ou ne se lie pas spécifiquement à une version tronquée de ladite séquence d'acides aminés C-terminale qui est QETCIQ (SEQ ID NO : 3).
